# EUROPÄISCHE PATENTANMELDUNG

(11) **EP 1 913 873 A1**
(43) Veröffentlichungstag der Anmeldung: **23.04.2008**
(21) Anmeldenummer: 07117861.0
(22) Anmeldetag: 04.10.2007
(51) Int. Cl.: A61B 6/00, A61B 5/06, A61B 6/12, A61B 19/00

(54) **Erfassungsvorrichtung zum Erfassen eines Objekts in bis zu drei Dimensionen mittels Röntgenstrahlen in zueinander verschiedenen Erfassungsrichtungen**

(30) Priorität: 20.10.2006 DE 102006049575
(71) Anmelder: SIEMENS AKTIENGESELLSCHAFT, 80333 München (DE)
(72) Erfinder: Klingenbeck-Regn, Klaus, 90429, Nürnberg (DE)

(57) **Zusammenfassung**

Die Erfindung betrifft eine Erfassungsvorrichtung zum Erfassen eines Objekts in bis zu drei Dimensionen mit einem Röntgensender und einem in einer Erfassungsebene angeordneten Detektor für die Röntgenstrahlen, welche die Röntgenstrahlen erfassen und wenigstens einen 2D-Datensatz erzeugen, welcher das Objekt in einer Projektion repräsentiert. Die Erfassungsvorrichtung weist auch einen mit dem Röntgensender und mit dem Detektor verbunden C-Bogen und eine mit dem C-Bogen wirkverbundene Stellvorrichtung auf, welche ausgebildet ist, den C-Bogen in Abhängigkeit von einem eingangsseitig empfangenen Stellsignal in wenigstens zwei rotatorischen Freiheitsgraden zu bewegen und in einer durch das Stellsignal repräsentierten Erfassungsposition festzustellen. Die Erfassungsvorrichtung ist ausgebildet, einen 3D-Datensatz zu erzeugen, welcher das Objekt in drei Dimensionen repräsentiert und diesen zusammen mit dem wenigstens einen 2D-Datensatz zum Wiedergeben mittels wenigstens eine Bildwiedergabeeinheit auszugeben. Die Erfassungsvorrichtung weist einen Positionsspeicher für Positionsdatensätze auf, welche jeweils eine Erfassungsposition repräsentieren und kann in Abhängigkeit von einem Benutzerinteraktionssignal wenigstens einen Positionsdatensatz aus dem Positionsspeicher auslesen und ein dem Positionsdatensatz entsprechendes, eine Erfassungsposition repräsentierendes Stellsignal zum Bewegen des C-Bogens in die Erfassungsposition zu erzeugen und dort einen 2D-Datensatz mittels des Detektors erzeugen.

## Beschreibung

Die Erfindung betrifft eine Erfassungsvorrichtung zum Erfassen eines Objekts in bis zu drei Dimensionen. Die Erfassungsvorrichtung weist einen Röntgensender auf, welcher ausgebildet ist, Röntgenstrahlen auszusenden. Die Erfassungsvorrichtung weist auch einen in einer Erfassungsebene angeordneten Detektor für die Röntgenstrahlen auf, welcher derart angeordnet und ausgebildet ist, die Röntgenstrahlen zu erfassen und wenigstens einen 2D-Datensatz zu erzeugen, welcher das Objekt in einer Projektion durch das Objekt hindurch auf die Erfassungsebene repräsentiert. Die Erfassungsvorrichtung ist ausgebildet, einen das Objekt in drei räumlichen Dimensionen repräsentierenden 3D-Datensatz, insbesondere mittels Rückprojektion, aus einer Mehrzahl von 2D-Datensätzen zu erzeugen, welche das Objekt jeweils in zueinander verschiedenen Erfassungsrichtungen in einer Projektion durch das Objekt hindurch repräsentieren und den 3D-Datensatz in einem Speicher vorrätig zu halten. Die Erfassungsvorrichtung weist auch einen C-Bogen auf, welcher mit dem Röntgensender und mit dem Detektor verbunden ist. Die Erfassungsvorrichtung weist eine mit dem C-Bogen wirkverbundene Stellvorrichtung auf, welche ausgebildet ist, den C-Bogen in Abhängigkeit von einem eingangsseitig empfangenen Stellsignal in wenigstens zwei oder drei rotatorischen Freiheitsgraden zu bewegen und in einer durch das Stellsignal repräsentierten Erfassungsposition festzustellen. Die Erfassungsvorrichtung ist ausgebildet, aus dem 3D-Datensatz einen Bild-Datensatz zu erzeugen, welcher das Objekt, insbesondere eine Aufsicht auf das Objekt, eine Durchsicht durch das Objekt oder einen Schnitt durch das Objekt repräsentiert und diesen zusammen mit dem wenigstens einen 2D-Datensatz zum Wiedergeben mittels wenigstens einer Bildwiedergabeeinheit auszugeben.

Die der Erfindung zugrunde liegende Aufgabe ist es, eine Erfassungsvorrichtung mit einem verbesserten Bedienungskomfort anzugeben.

Diese Aufgabe wird durch eine Erfassungsvorrichtung der eingangsgenannten Art gelöst. Die Erfassungsvorrichtung weist einen Positionsspeicher für Positionsdatensätze auf, welche jeweils eine Erfassungsposition repräsentieren. Die Erfassungsvorrichtung ist ausgebildet, in Abhängigkeit von einem Benutzerinteraktionssignal wenigstens einen Positionsdatensatz aus dem Positionsspeicher auszulesen und ein dem Positionsdatensatz entsprechendes, eine Erfassungsposition repräsentierendes Stellsignal zum Bewegen des C-Bogens in die Erfassungsposition zu erzeugen und dort einen 2D-Datensatz mittels des Detektors zu erzeugen.

Durch die erfindungsgemäße Erfassungsvorrichtung kann ein Benutzer der Erfassungsvorrichtung vorteilhaft eine für eine Intervention vorgesehene, vorbestimmte Sequenz von Erfassungspositionen anfahren und in den Erfassungspositionen jeweils wenigstens einen 2D-Datensatz erzeugen. Der Benutzer kann das durch den 2D-Datensatz repräsentierte Objekt vorteilhaft auf einer Bildwiedergabeeinheit zusammen mit dem 3D-Datensatz betrachten und so die Intervention - in-vivo - mittels des wenigstens einen 2D-Datensatzes verfolgen und gleichzeitig eine Aufsicht, eine Durchsicht oder einen Schnitt durch das durch den 3D-Datensatz repräsentierte Objekt betrachten.
Die Erfassungsvorrichtung kann zum Vorrätighalten des 3D-Datensatzes in einem Speicher einen Anschluß für einen Speicher, insbesondere einen Datenbus, oder den Speicher aufweisen.

In einer bevorzugten Ausführungsform weist die Erfassungsvorrichtung einen Ortsensor auf, welcher ausgebildet ist, einen Instrumentort eines medizinischen Instruments in einem für das Erfassen des Objekts vorgesehene räumliche Bereich zu erfassen und einen dem Instrumentort repräsentierenden Instrument-Datensatz zu erzeugen und diesen einem dem Instrumentort entsprechenden Bereich des 3D-Datensatzes zuzuordnen. Die Erfassungsvorrichtung ist ausgebildet, den Bild-Datensatz in Abhängigkeit von dem Instrument-Datensatz derart zu erzeugen, dass der Bild-Datensatz zusätzlich das medizinische Instrument repräsentiert.

Durch den Ortsensor kann ein Benutzer eine Position eines medizinischen Instruments während einer Intervention innerhalb eines durch den 3D-Datensatzes repräsentierten, mittels einer Bildwiedergabe wiedergegebenen, Objekts beobachten.

In einer vorteilhaften Ausführungsform ist der Ortsensor ein elektromagnetischer Ortsensor, welcher ausgebildet ist, einen Instrumentort mittels wenigstens zwei, bevorzugt drei zueinander verschieden ausgerichteten elektromagnetischen Feldern zu erfassen und einen Instrument-Datensatz zu erzeugen, welcher den Instrumentort repräsentiert.

In einer anderen Ausführungsform ist der Ortsensor ein Ultraschall-Ortsensor, welcher ausgebildet ist, mittels zwei, mit dem Instrument verbundenen, zueinander beabstandeten Ultraschallsendern und drei, zu den Ultraschallsendern räumlich beabstandeten Ultraschallempfängern, beispielsweise Elektret-Kondensator-Mikrophone, in Abhängigkeit von einer LaufzeitDifferenz von den Ultraschallsendern erzeugter UltraschallSignale einen räumlichen Instrumentort des medizinischen Instruments zu erfassen und einen entsprechenden Instrument-Datensatz zu erzeugen, welcher den Instrumentort repräsentiert.

In einer anderen Ausführungsform ist der Ortsensor ausgebildet, eine räumliche Ausrichtung eines magnetisierbaren oder eines permanentmagnetischen Objekts, insbesondere aus zwei, bevorzugt aus drei zueinander verschiedenen Erfassungsrichtungen zu erfassen und in Abhängigkeit von der räumlichen Ausrichtung des magnetisierbaren oder permanentmagnetischen Objekts einen räumlichen Ort des magnetisierbaren oder permanentmagnetischen Objekts zu erfassen. Das magnetisierbare oder permanentmagnetische Objekt kann beispielsweise mit dem medizinischen Instrument, insbesondere im Bereich eines Katheterendes oder im Bereich eines Endes eines Führungsdrahtes oder eines anderen medizinischen oder chirurgischen Instruments, verbunden sein. Der Ortsensor ist in dieser Ausführungsform ausgebildet, einen Instrument-Datensatz zu erzeugen, welcher den Ort des magnetisierbaren oder permanentmagnetischen Objekts repräsentiert.

In einer anderen Ausführungsform ist der Ortsensor ein optischer Ortsensor, welcher mittels elektromagnetischer Strahlen, insbesondere im infraroten Wellenlängenbereich, einen Instrumentort des Instruments, insbesondere interferometrisch erfassen kann und einen den Instrumentort repräsentierenden Instrument-Datensatz erzeugen kann.

In einer bevorzugten Ausführungsform weist die Erfassungsvorrichtung einen Koordinatenspeicher auf und ist ausgebildet, einen wenigstens einen Erfassungsort des 2D-Datensatzes repräsentierenden Objekt-Koordinaten-Datensatz zu erzeugen und den Objekt-Koordinaten-Datensatz in dem Koordinatenspeicher abzuspeichern. Die Erfassungsvorrichtung ist weiter ausgebildet, den in dem Koordinatenspeicher abgespeicherten Objekt-Koordinaten-Datensatz auszulesen und den Instrumentort im Verhältnis zu dem ausgelesenen Objekt-Koordinaten-Datensatz, oder in Form von Objekt-Koordinaten auszugeben.

In einer bevorzugten Ausführungsform weist die Erfassungsvorrichtung eine Bildverarbeitungseinheit auf, welche ausgebildet ist, aus dem 3D-Datensatz einen 2D-Datensatz zu erzeugen, welcher eine Projektion durch das durch den 3D-Datensatz repräsentierte Objekt hindurch, insbesondere auf eine virtuelle Erfassungsebene, repräsentiert und diesen ausgangsseitig auszugeben. Durch die Bildverarbeitungseinheit kann vorteilhaft ein virtuelles Projektionsergebnis auf eine virtuelle Erfassungsebene aus einer beliebigen Erfassungsrichtung erzeugt werden, welche beispielsweise mittels eines zweiten Detektors und eines zweiten Röntgensenders, welche mittels eines C-Bogens verbunden sein können, nicht möglich ist. In einer vorteilhaften Ausführungsform kann die Bildverarbeitungseinheit den 3D-Datensatz aus den 2D-Datensätzen erzeugen.

In einer vorteilhaften Ausführungsform ist die Erfassungsvorrichtung ausgebildet, mittels des Detektors eine zeitliche Folge von 2D-Datensätzen zu erzeugen, welche jeweils zeitlich aufeinander folgende Erfassungsergebnisse des Objekts repräsentieren. Dadurch kann ein Benutzer der Erfassungsvorrichtung vorteilhaft ein für eine Intervention vorgesehenes Objekt in-vivo betrachten.

In einer bevorzugten Ausführungsform ist die Erfassungsvorrichtung, insbesondere die Bildverarbeitungseinheit ausgebildet, wenigstens zwei 2D-Datensätze für entsprechende Erfassungsorte voneinander zu subtrahieren. Dadurch kann vorteilhaft bei einem fluoroskopischen Erfassen eines Objekts ein mittels eines Röntgenkontrastmittels hervorgehobener Bereich, beispielsweise ein Gefäßsystem, insbesondere eines Herzens extrahiert werden oder ein Bildkontrast verbessert werden. Die Erfassungsvorrichtung kann dazu weiter ausgebildet sein, einen Angio-2D-Datensatz zu erzeugen, welcher ein Subtraktionsergebnis der Subtraktion repräsentiert.

In einer bevorzugten Ausführungsform weist die Erfassungsvorrichtung einen Bewegungssensor auf, welcher ausgebildet ist, ein Objektbewegen des Objekts zu erfassen und ein Bewegungssignal zu erzeugen, welches das Objektbewegen repräsentiert. Die Erfassungsvorrichtung ist auch ausgebildet, in Abhängigkeit von dem Bewegungssignal einen 3D-Datensatz zu erzeugen oder einen 2D-Datensatz aus dem 3D-Datensatz zu erzeugen. Der Bewegungssensor kann beispielsweise ein mit dem Objekt verbindbar ausgebildeter Beschleunigungsaufnehmer sein oder ein interferomerisch-optischer Bewegungssensor sein, welcher das Objektbewegen berührungslos erfassen kann.
Durch den Bewegungssensor kann vorteilhaft ein Objektbewegen erfasst werden, und in Abhängigkeit von dem Objektbewegen ein eine einer Objektbewegung entsprechender 2D-Datensatz aus dem 3D-Datensatz, repräsentierend eine Ansicht, Aufsicht, Durchsicht oder ein Schnitt durch das durch den 3D-Datensatz repräsentierte Objekt erzeugt werden und erneut mittels der Bildwiedergabeeinheit wiedergegeben werden.

In einer vorteilhaften Ausführungsform kann die Erfassungsvorrichtung dazu eine Korrelationseinheit aufweisen, welche ausgebildet ist, in Abhängigkeit eines Ähnlichkeitsparameters, insbesondere mittels Kreuzkorrelation, eine dem Objektbewegen entsprechende Ansicht, Durchsicht oder Aufsicht aus dem 3D-Datensatz zu ermitteln und einen entsprechenden Bild-Datensatz zu erzeugen.
In einer bevorzugten Ausführungsform der Erfassungsvorrichtung ist die Stellvorrichtung ausgebildet, den C-Bogen in Abhängigkeit von dem Stellsignal in wenigstens zwei oder drei translatorischen Freiheitsgraden zu bewegen und in einer durch das Stellsignal repräsentierten Erfassungsposition festzustellen. Dadurch kann ein Patiententisch vorteilhaft an eine für eine Intervention vorgesehene Position fixiert bleiben, so dass ein Benutzer sich während einer Intervention nicht fortbewegen muss.

Die Erfindung betrifft auch ein Verfahren zum Erfassen eines Objekts in bis zu drei Dimensionen mittels Röntgenstrahlen, bei welchem mittels eines Detektors für die Röntgenstrahlen eine Mehrzahl von 2D-Datensätzen erzeugt werden, wobei die 2D-Datensätze das Objekt jeweils in zueinander verschiedenen Erfassungsrichtungen in einer Projektion durch das Objekt hindurch repräsentieren und ein das Objekt in drei räumlichen Dimensionen repräsentierender 3D-Datensatz aus den 2D-Datensätzen erzeugt wird, welcher das Objekt in drei Dimensionen repräsentiert.
Bei dem Verfahren wird aus dem 3D-Datensatz ein Bild-Datensatz erzeugt, welcher das Objekt, insbesondere eine Aufsicht auf das Objekt, eine Durchsicht durch das Objekt oder einen Schnitt durch das Objekt repräsentiert und dieser zusammen mit wenigstens einem von dem Detektor, insbesondere in-vivo, erzeugtem, das Objekt in einer Projektion durch das Objekt hindurch repräsentierenden 2D-Datensatz oder einer zeitlichen Folge von 2D-Datensätzen mittels einer Bildwiedergabeeinheit wiedergegeben wird. Weiter werden wenigstens zwei Positionsdatensätze vorrätig gehalten, welche jeweils zueinander verschiedene Erfassungspositionen zum Erzeugen eines 2D-Datensatzes repräsentieren und für die Positionsdatensätze - insbesondere für einen Teil der Positionsdatensätze oder für jeden Positionsdatensatz - erfolgt ein Erfassen des Objekts in Abhängigkeit von einem Benutzerinteraktionssignal entsprechend der von den Positionsdatensätzen jeweils repräsentierten Erfassungsposition. Weiter wird in der Erfassungsposition mittels des Detektors, insbesondere in-vivo, der 2D-Datensatz oder die zeitliche Folge von 2D-Datensätzen erzeugt.
Durch das Verfahren kann ein Benutzer, insbesondere ein Arzt während einer Intervention vorteilhaft ein Objekt in drei Dimensionen erfassen, ein entsprechendes 3D-Erfassungsergebnis erzeugen und während einer folgenden Intervention zueinander verschiedene 2D-Erfassungsergebnisse fluoroskopisch, nämlich in-vivo, erzeugen und diese zusammen mit dem 3D-Erfassungsergebnis auf einer Bildwiedergabeeinheit betrachten.

In einer vorteilhaften Ausführungsform des Verfahrens kann aus wenigstens zwei 2D-Datensätzen mittels Subtraktion für entsprechende Erfassungsorte ein Subtraktionsergebnis erzeugt werden und ein dieses repräsentierender Angio-2D-Datensatz erzeugt und dieser zusammen mit dem Bild-Datensatz mittels der Bildwiedergabeeinheit wiedergegeben werden.

Dadurch kann ein Benutzer beispielsweise durch Verabreichen eines Kontrastmittels ein Erfassungsergebnis erzeugen, welches einen Gefäßbaum des Objekts repräsentiert.

In einer vorteilhaften Ausführungsform des Verfahrens wird ein Instrumentort eines medizinischen Instruments in einem für das Erfassen des Objekts vorgesehenen räumlichen Bereich erfasst, und ein den Instrumentort repräsentierenden Instrument-Datensatz erzeugt und einem dem Instrumentort entsprechenden Bereich des 3D-Datensatzes zugeordnet,
und aus dem 3D-Datensatz ein Bild-Datensatz erzeugt, welcher das Objekt, insbesondere eine Aufsicht auf das Objekt, eine Durchsicht durch das Objekt oder einen Schnitt durch das Objekt, zusammen mit dem Instrument repräsentiert.

Dadurch kann ein Benutzer ein medizinisches Instrument, beispielsweise einen Katheter, insbesondere einen Ablationskatheter, einen Führungsdraht oder ein Hochfrequenzchirurgisches Instrument zusammen mit dem 3D-Erfassungsergebnis betrachten, wobei das medizinische Instrument in den in-vivo erfassten 3D-Erfassungseregnissen mit erfasst worden ist - und so ebenfalls in dem 2D-Erfassungsergebnis repräsentiert ist.

Die Erfindung wird nun im Folgenden anhand von Figuren und weiteren Ausführungsbeispielen erläutert.
Figur 1 zeigt schematisch ein Ausführungsbeispiel für eine Erfassungsvorrichtung zum Erfassen eines Objekts mittels Röntgenstrahlen mit einem Röntgensender und einem Detektor;
Figur 2 zeigt schematisch ein Ausführungsbeispiel für einen C-Bogen;
Figur 3 zeigt ein Ausführungsbeispiel für ein Verfahren zum Erfassen einem Objekts mittels Röntgenstrahlen.

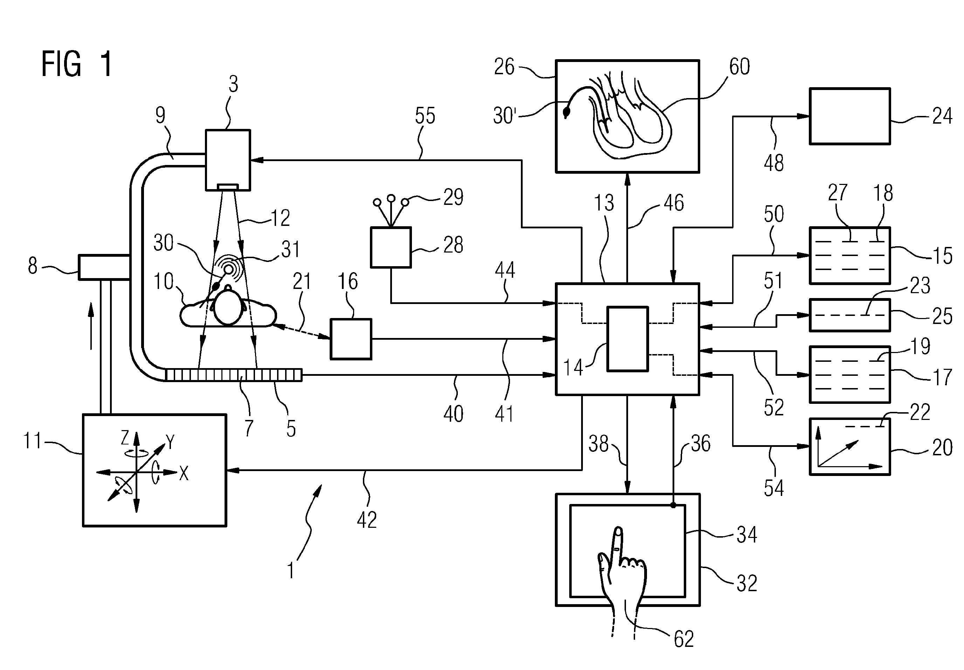

Figur 1 zeigt schematisch ein Ausführungsbeispiel für eine Erfassungsvorrichtung 1 mit einem Röntgensender 3 und einem Detektor 5. Der Detektor 5 weist eine Vielzahl von Detektormatrixelementen auf, von denen das Detektormatrixelement 7 beispielhaft bezeichnet ist. Der Röntgensender 3 ist mittels eines C-Bogens 9 mit dem Detektor 5 derart verbunden, dass ein Objekt 10 mittels von dem Röntgensender 3 erzeugter Röntgenstrahlen 12 in einer Projektion durch das Objekt 10 hindurch auf den Detektor 5 erfasst werden kann. Der C-Bogen 9 ist schwenkbar gelagert und kann in drei rotatorischen Freiheitsgraden geschwenkt werden, insbesondere um eine Achse X, eine Achse Y oder eine Achse Z. Die Achsen X, Y und Z bilden zusammen ein Orthogonalsystem. Der C-Bogen kann auch in drei translatorischen Freiheitsgraden bewegt werden, insbesondere parallel zu der Achse X, parallel zu der Achse Y oder parallel zu der Achse Z. Dazu ist der C-Bogen 9 mittels einer Stellmechanik 8 mit einer Stellvorrichtung 11 derart verbunden, dass der C-Bogen 9 rotatorisch und/oder translatorisch bewegt werden kann. Dazu ist die Stellvorrichtung ausgebildet, den C-Bogen 9 in Abhängigkeit von einem eingangsseitig empfangenen Stellsignal mittels der Stellmechanik 8 zu bewegen und in einer durch das Stellsignal repräsentierten Erfassungsposition festzustellen.

Die Detektormatrixelemente des Detektors 5 sind jeweils ausgebildet, Röntgenstrahlen zu empfangen und in Abhängigkeit der empfangenen Röntgenstrahlen ein Detektormatrixelementsignal zu erzeugen, welches eine Strahlintensität des empfangenen Röntgenstrahls repräsentiert. Die Detektormatrixelemente können jeweils Selen oder Silizium, insbesondere amorphes Silizium aufweisen. Die Erfassungsvorrichtung 1 weist auch eine zentrale Verarbeitungseinheit 13 auf. Die zentrale Verarbeitungseinheit 13 weist eine Zuordnungseinheit 14 auf. Die Erfassungsvorrichtung 1 weist auch einen Speicher 15 und einen Speicher 17 auf. Der Speicher 15 ist zum Vorrätighalten von 2D-Datensätzen ausgebildet, von denen der 2D-Datensatz 18 beispielhaft dargestellt ist. Der Speicher 17 ist zum Vorrätighalten von wenigstens einem 3D-Datensatz ausgebildet, von denen der 3D-Datensatz 19 beispielhaft dargestellt ist.

Die Erfassungsvorrichtung 1 weist auch einen Positionsspeicher 25 auf, welcher zum Vorrätighalten von Positionsdatensätzen ausgebildet ist, welche jeweils eine Erfassungsposition repräsentieren. Der Positionsdatensatz 23 ist beispielhaft bezeichnet.

Die Erfassungsvorrichtung 1 weist auch einen Koordinatenspeicher 20 auf, welcher ausgebildet ist, einen Objekt-Koordinaten-Datensatz vorrätig zu halten, wobei der Objekt-Koordinaten-Datensatz 22 beispielhaft bezeichnet ist. Der Speicher 15, der Speicher 17 und der Speicher 20 können zusammen durch einen gemeinsamen Speicher verwirklicht sein. Die Speicher 15, 17, 20 und 25 sind jeweils als Schreib-LeseSpeicher, insbesondere als nichtflüchtige Schreib-LeseSpeicher ausgebildet.

Die Erfassungsvorrichtung 1 weist auch eine Bildverarbeitungseinheit 24 auf. Die Bildverarbeitungseinheit 24 ist ausgebildet, aus einer Mehrzahl von 2D-Datensätzen, welche jeweils ein Erfassungsergebnis einer Projektion von Röntgenstrahlen 12 durch das Objekt 10 hindurch aus jeweils zueinander verschiedenen Erfassungsrichtungen repräsentieren - dazu kann beispielsweise der Röntgensender 3 zusammen mit dem Detektor 5 und dem C-Bogen 9 mittels der Stellvorrichtung 11 um das Objekt 10 herum geschwenkt worden sein - ein 3D-Datensatz erzeugen, welcher das Objekt 10 in drei Dimensionen repräsentiert. Der 3D-Datensatz kann beispielsweise mittels Rückprojektion, insbesondere gefilterter Rückprojektion durch die Bildverarbeitungseinheit 24 erzeugt werden. Der 3D-Datensatz kann eine Vielzahl von Voxel-Objektpunkten repräsentieren, welche zusammen das Objekt 10 in drei Dimensionen repräsentieren.

Die Erfassungsvorrichtung 1 weist auch eine Bildwiedergabeeinheit 26 auf. Die Erfassungsvorrichtung 1 weist auch eine Eingabeeinheit 32 mit einer berührungsempfindlichen Oberfläche 34 auf. Die Eingabeeinheit 32 weist in dieser Ausführungsform eine Bildwiedergabeeinheit mit der berührungsempfindlichen Oberfläche 34 auf. Die berührungsempfindliche Oberfläche 34 ist ausgebildet, in Abhängigkeit von einem Berühren - durch eine Benutzerhand 62 - ein Benutzerinteraktionssignal zu erzeugen, welches den Ort des Berührens der berührungsempfindlichen Oberfläche 34 repräsentiert und dieses ausgangsseitig auszugeben. Die Erfassungsvorrichtung 1 weist auch einen Ortsensor 28 auf. Der Ortsensor 28 weist wenigstens eine Antenne 29 auf, welche ausgebildet ist, ein elektromagnetisches Feld 31 des medizinischen Instruments 30 zu erfassen. Das medizinische Instrument 30 ist ausgebildet, das elektromagnetische Feld 31 zu erzeugen. Der Ortsensor 28 ist ausgebildet, in Abhängigkeit von dem erfassten elektromagnetischen Feld 31 einen Instrument-Datensatz zu erzeugen, welcher den Instrumentort des Instruments 30 repräsentiert und diesen ausgangsseitig auszugeben. Die berührungsempfindliche Oberfläche 34 ist ausgangsseitig über eine Verbindungsleitung 36 mit der zentralen Verarbeitungseinheit 13 verbunden. Die zentrale Verarbeitungseinheit 13 ist über eine Verbindungsleitung 38 mit der Eingabeeinheit 32 und dort mit der Bildwiedergabeeinheit der Eingabeeinheit 32 verbunden. Der Detektor 5 ist ausgangsseitig über eine Verbindungsleitung 40 mit der zentralen Verarbeitungseinheit 13 verbunden. Die zentrale Verarbeitungseinheit 13 ist ausgangsseitig über eine Verbindungsleitung 42 mit der Schwenkvorrichtung 11 verbunden. Die zentrale Verarbeitungseinheit 13 ist eingangsseitig über eine Verbindungsleitung 44 mit dem Ortsensor 28, über eine Verbindungsleitung 46 mit der Bildwiedergabeeinheit 26, über eine Verbindungsleitung 48 mit der Bildverarbeitungseinheit 24, über eine Verbindungsleitung 50 mit der Speichereinheit 15, über eine Verbindungsleitung 52 mit der Speichereinheit 17 und über eine Verbindungsleitung 54 mit dem Koordinatenspeicher 20 verbunden. Die Erfassungsvorrichtung weist auch einen Bewegungssensor 16 auf, welcher mittels eines optischen Strahls 21 - beispielsweise eines elektromagnetischen Strahls im infraroten Wellenlängenbereich - ein Objektbewegen insbesondere interferometrisch erfassen kann und ein das Objektbewegen repräsentierendes Bewegungssignal erzeugen kann. Der Bewegungssensor 16 ist ausgangsseitig über eine Verbindungsleitung 41 mit der zentralen Verarbeitungseinheit 13 verbunden. Die Verbindungsleitungen 48, 50, 51, 52 oder 54 können sind jeweils bidirektional ausgebildet und können jeweils ein Datenbus sein.

Die Funktionsweise der Erfassungsvorrichtung 1 wird nun im Folgenden erläutert:

Die zentrale Verarbeitungseinheit 13 ist ausgebildet, in Abhängigkeit von einem eingangsseitig über die Verbindungsleitung 36 empfangenen Benutzerinteraktionssignal ein Steuersignal zum Erzeugen des Röntgenstrahls 12 mittels des Röntgensenders 3 erzeugen und dieses ausgangsseitig über die Verbindungsleitung 55 ausgeben. Das Steuersignal zum Erzeugen des Röntgenstrahls 12 kann beispielsweise eine Beschleunigungsspannung, eine Bestrahlungszeit oder eine die Röntgenstrahlen 12 erzeugende elektrische Ladungsmenge repräsentieren. Der Detektor 5 kann die vom Röntgensender 3 erzeugten Röntgenstrahlen 12 durch das Objekt 10 hindurch in einer Projektion auf eine Erfassungsebene erfassen, in welcher der Detektor 5 angeordnet ist und einen 2D-Datensatz erzeugen, welcher das Objekt 10 in einer Projektion durch das Objekt 10 hindurch auf die Erfassungsebene repräsentiert. Der 2D-Datensatz repräsentiert dabei eine 2D-Matrix, gebildet aus Matrixelementen, welche jeweils einen Intensitätswert repräsentieren, der dem entsprechend zugeordneten Detektormatrixelementsignal eines Detektormatrixelements entspricht. Die zentrale Verarbeitungseinheit 13 kann den 2D-Datensatz über die Verbindungsleitung 40 eingangsseitig empfangen und über die Verbindungsleitung 50 in dem Speicher 15 abspeichern. Dort ist der 2D-Datensatz 18 beispielhaft bezeichnet.

Die zentrale Verarbeitungseinheit 13 kann zum Erzeugen weiterer 2D-Datensätze, welche das Objekt 10 in zueinander verschiedenen Erfassungsrichtungen repräsentieren, - beispielsweise in Abhängigkeit von einem über die Verbindungsleitung 36 empfangenen Benutzerinteraktionssignal - aus dem Positionsspeicher 25 wenigstens einen Positionsdatensatz auslesen und ein dem Positionsdatensatz entsprechendes, eine Erfassungsposition repräsentierendes Stellsignal erzeugen und dieses ausgangsseitig über die Verbindungsleitung 42 an die Stellvorrichtung 11 senden. Die Stellvorrichtung 11 kann in Abhängigkeit von dem Stellsignal den C-Bogen 9 zusammen mit dem Detektor 5 und dem Röntgensender 3 um das Objekt 10 herum - gemäß der drei rotatorischen und der drei translatorischen Freiheitsgrade - in die dem Stellsignal entsprechende Erfassungsposition fahren und dort fixieren.

In einem weiteren Interventionsverlauf kann der C-Bogen 9 entsprechend einem weiteren Positionsdatensatz in eine weitere Erfassungsposition wie vorab beschrieben gefahren werden. Die zentrale Verarbeitungseinheit 13 kann dann ein weiteres Signal zum Erzeugen eines Röntgenstrahls 12 über die Verbindungsleitung 55 an den Röntgensender 3 senden und ein von dem Detektor 5 erzeugtes Erfassungsergebnis, nämlich wenigstens einen 2D-Datensatz über die Verbindungsleitung 40 empfangen und über die Verbindungsleitung 50 in dem Speicher 15 abspeichern. Die zentrale Verarbeitungseinheit 13 kann auf diese Weise eine Mehrzahl von 2D-Datensätzen erzeugen, welche jeweils das Objekt 10 in einer Projektion durch das Objekt hindurch auf eine Erfassungsebene mit jeweils zueinander verschiedenen Erfassungsrichtungen repräsentieren. Die zentrale Verarbeitungseinheit 13 kann nun - beispielsweise in Abhängigkeit von einem über die Verbindungsleitung 36 empfangenen Benutzerinteraktionssignal - die 2D-Datensätze aus dem Speicher 15 über die Verbindungsleitung 50 auslesen und über die Verbindungsleitung 48 an die Bildverarbeitungseinheit 24 senden.

Die Bildverarbeitungseinheit 24 kann aus den empfangenen 2D-Datensätzen einen 3D-Datensatz erzeugen, beispielsweise mittels eines Rückprojektionsalgorithmus, insbesondere eines filternden Rückprojektionsalgorithmus. Die Bildverarbeitungseinheit 24 kann den so erzeugten 3D-Datensatz, welcher das Objekt 10 in drei Dimensionen repräsentiert und über die Verbindungsleitung 48 an die zentrale Verarbeitungseinheit 13 zurücksenden. Der 3D-Datensatz kann eine Vielzahl von Voxel-Objektpunkten repräsentieren, welche jeweils einen Wert eines Absorptionskoeffizienten für Röntgenstrahlen an einem Objektort repräsentieren und somit zusammen das Objekt 10 in drei Dimensionen repräsentieren. Die zentrale Verarbeitungseinheit 13 kann den über die Verbindungsleitung 48 empfangenen 3D-Datensatz über die Verbindungsleitung 52 in dem Speicher 17 abspeichern. Dort ist der 3D-Datensatz 19 beispielhaft bezeichnet. Die zentrale Verarbeitungseinheit 13 kann über die Verbindungsleitung 44 eingangsseitig einen Instrument-Datensatz empfangen, welcher einen Instrumentort des Instruments 30 repräsentiert. Das Instrument 30 ist in diesem Ausführungsbeispiel innerhalb des Objekts 10 angeordnet. Die zentrale Verarbeitungseinheit 13 kann beispielsweise zum Kalibrieren der Erfassungsvorrichtung 1 über die Verbindungsleitung 44 einen Instrument-Datensatz empfangen und wenigstens einen Erfassungsort des 3D-Datensatzes repräsentierenden Objekt-Koordinaten-Datensatz erzeugen und diesen über die Verbindungsleitung 44 an den Koordinatenspeicher 20 zu senden und dort abzuspeichern. Der Objekt-Koordinaten-Datensatz 22 ist beispielhaft bezeichnet und repräsentiert entweder wenigstens zwei Erfassungsorte, jeweils für ein Voxel des 3D-Datensatzes, oder einen Erfassungsort für ein Voxel und eine räumliche Ausrichtung, beispielsweise in Form eines Vektors, welcher eine Ausrichtung des 3D-Datensatzes repräsentiert.

Anders als vorab beschrieben kann vor einem Auslesen des Positionsspeichers 25 bereits ein 3D-Datzensatz aus einer Mehrzahl von 2D-Datensätzen erzeugt werden und in dem Speicher 17 abgespeichert werden. Anschließend kann ein Benutzer - beispielsweise mit der Benutzerhand 62 - ein Benutzerinteraktionssignal zum Auslesen eines Positionsdatensatzes aus dem Positionsspeicher 25 und zum Anfahren einer weiteren Erfassungsposition erzeugen.

Die zentrale Verarbeitungseinheit 13 kann beispielsweise ein Benutzermenüsignal erzeugen und dieses über die Verbindungsleitung 38 an die Eingabeeinheit 32 senden, Das Benutzermenüsignal kann die in dem Speicher 25 vorrätig gehaltenen Erfassungspositionen, insbesondere alphanumerisch oder in Form von grafischen Symbolen, repräsentieren. Der Benutzer kann ein einer Erfassungsposition entsprechendes Benutzerinteraktionssignal erzeugen und dieses über die Verbindungsleitung 36 an die zentrale Verarbeitungseinheit 13 senden. Die zentrale Vererbeitungseinheit kann für die entsprechende Erfassungsposition ein Stellsignal erzeugen und dieses an die Stellvorrichtung 11 senden und einen 2D-Datensatz mittels des Röntgensenders 3 und des Detektors 5 erzeugen und in dem Speicher 15 abspeichern.

Die Erfassungsvorrichtung 1 kann beispielsweise bei einer Erfassungsposition - in-vivo - einen fluoroskopischen 2D-Datensatz oder ein zeitliche Folge von 2D-Datensätzen erzeugen. Die Erfassungsvorrichtung kann beispielsweise mittels der Bildverarbeitungseinheit 24 einen Angio-2D-Datensatz erzeugen, welcher ein Gefäßsystem des erfassten Objekts 10 repräsentiert. Dazu kann die Bildverarbeitungseinheit 24 wenigstens zwei 2D-Datensätze voneinander für jeden Erfassungsort, insbesondere für jedes Matrixelement einer durch den 2D-Datensatz repräsentierten Matrix - subtrahieren und als Subtraktionsergebnis den Angio-2D-Datensatz erzeugen. Der Angio-2D-Datensatz 27 ist beispielhaft bezeichnet. So kann die Erfassungsvorrichtung einen mittels eines Kontrastmittels erzeugten Bildkontrast erhöhen.

Während einer Intervention kann die zentrale Verarbeitungseinheit, insbesondere eine Zuordnungseinheit 14 einen über die Verbindungsleitung 44 empfangenen Instrument-Datensatz einem durch einen Teil des 3D-Datensatzes repräsentierten Objektort zuordnen und ein Zuordnungsergebnis erzeugen, welches dem Instrumentort innerhalb des durch den 3D-Datensatz repräsentierten Raumes entspricht. Die zentrale Verarbeitungseinheit 13 kann, beispielsweise mittels des von der Zuordnungseinheit 14 erzeugten Zuordnungsergebnisses, einen Bild-Datensatz erzeugen, welcher das Objekt 10, insbesondere beispielsweise ein Herz 60 des Objekts 10 in drei Dimensionen zusammen mit dem Instrument 30 repräsentiert.

Die zentrale Verarbeitungseinheit 13 einen 3D-Datensatz beispielsweise auch aus Angio-2D-Datensätzen erzeugen, so dass der 3D-Datensatz ein Gefäßsystem des Objekts repräsentiert.

Die zentrale Verarbeitungseinheit kann während eines weiteren Interventionsverlaufs eine zeitliche Folge von 2D-Datensätzen oder Angio-2D-Datensätzen erzeugen und diese über die Verbindungsleitung 40 empfangen, in dem Speicher 15 vorrätighalten, und diese zum gemeinsamen Wiedergeben mit dem Bild-Datensatz mittels der Bildwiedergabeeinheit 26 wieder auslesen. Die Bildwiedergabeeinheit 26 gibt beispielhaft das Herz 60 und das Instrument 30' wieder. Das Objekt 10 kann beispielsweise bewegt worden sein, so dass ein erneutes Zuordnen erforderlich ist. Die zentrale Verarbeitungseinheit 13 kann dazu beispielsweise in Abhängigkeit eines über die Verbindungsleitung 41 empfangenen Bewegungssignals ein Erfassen des Objekts 10 zum Erzeugen eines 3D-Datensatzes erneut starten, oder in Abhängigkeit eines Ähnlichkeitsparameters, und insbesondere mittels der Bildverarbeitungseinheit 24, einen neuen 2D-Datensatz aus dem 3D-Datensatz erzeugen, welcher eine Durchsicht durch das Objekt 10 hindurch, eine Aufsicht oder einen Schnitt durch das Objekt 10 repräsentiert.

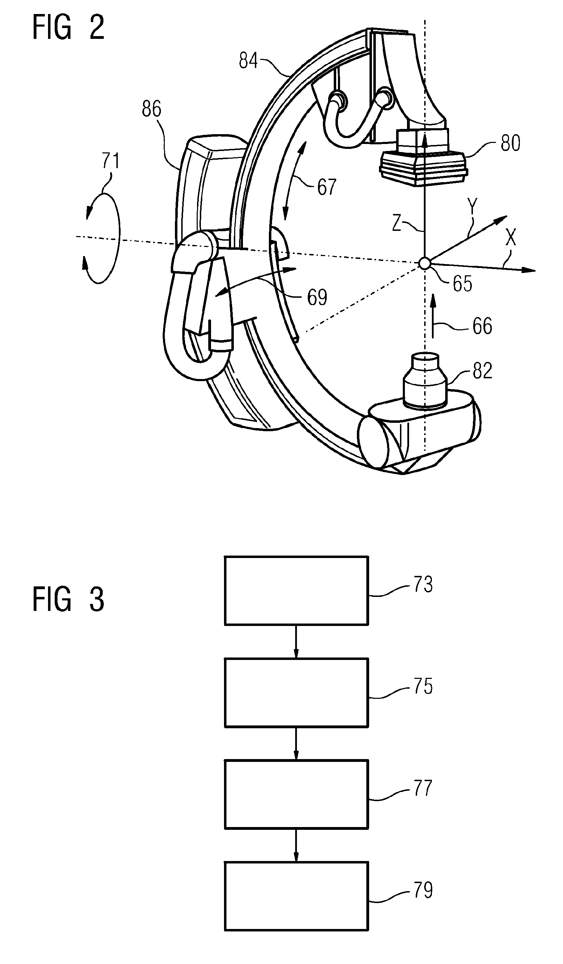

Figur 2 zeigt schematisch ein Ausführungsbeispiel für einen C-Bogen 84 - welcher beispielsweise anstelle des in Figur 1 dargestellten C-Bogens 9 Teil der Erfassungsvorrichtung 1 sein kann. Der C-Bogen 84 ist mit einer Stellvorrichtung 86 mindestens mittelbar verbunden. Der C-Bogen 84 weist einen Röntgensender 82 und einen Detektor 80 auf. Der Röntgensender 82 ist im Bereich eines ersten Endes des C-Bogens 84 und der Detektor 80 ist im Bereich eines zweiten Endes des C-Bogens 84 derart angeordnet, dass ein im Bereich eines Isozentrums 65 angeordnetes Objekt - beispielsweise das in Figur 1 dargestellte Objekt 10 - mittels eines von dem Röntgensender 82 entlang einer Erfassungsrichtung 66 ausgesendeten Röntgenstrahls durchstrahlt werden kann.

Der Detektor 80 ist derart angeordnet und ausgerichtet, den von dem Röntgensender 82 ausgesendeten Röntgenstrahl zu empfangen. Der C-Bogen 84 ist ausgebildet, geführt von der Stellvorrichtung 86, eine Translationsbewegung entlang einer Längsachse Y, entlang einer Querachse X, oder entlang einer Hochachse Z, oder entlang einer Kombination aus diesen Translationsachsen auszuführen.

Der C-Bogen 9 ist auch ausgebildet, geführt von der Stellvorrichtung 86, eine Schwenkbewegung entlang eines rotatorischen Freiheitsgrades 67, entlang eines rotatorischen Freiheitsgrades 69 oder entlang eines rotatorischen Freiheitsgrades 71 auszuführen. Ein Rotationsbewegen des C-Bogens 84 in dem rotatorischen Freiheitsgrad 67 oder in dem rotatorischen Freiheitsgrad 69 erfolgt dabei um eine Rotationsachse, welche durch das Isozentrum 65 verläuft.

Figur 3 zeigt ein Ausführungsbeispiel für ein Verfahren zum Erfassen einem Objekts mittels Röntgenstrahlen in bis zu drei Dimensionen.

In einem Schritt 73 werden Positionsdatensätze vorrätig gehalten, welche jeweils zueinander verschiedene Erfassungspositionen zum Erzeugen eines 2D-Datensatzes oder einer Folge von 2D-Datensätzen repräsentieren.

In einem Schritt 75 werden mittels eines Detektors für die Röntgenstrahlen eine Mehrzahl von 2D-Datensätzen erzeugt, wobei die 2D-Datensätze das Objekt jeweils in zueinander verschiedenen Erfassungsrichtungen in einer Projektion durch das Objekt hindurch repräsentieren, und ein das Objekt in drei räumlichen Dimensionen repräsentierender 3D-Datensatz aus den 2D-Datensätzen erzeugt, welcher das Objekt in drei Dimensionen repräsentiert.

In einem Schritt 77 erfolgt für jeden Positionsdatensatz ein Erfassen des Objekts in Abhängigkeit von einem Benutzerinteraktionssignal entsprechend der von den Positionsdatensätzen repräsentierten Positionen und für jede Erfassungsposition wird der 2D Datensatz oder die zeitliche Folge von 2D-Datensätzen erzeugt.

In einem Schritt 79 wird aus dem 3D-Datensatz ein Bild-Datensatz erzeugt wird, welcher das Objekt, insbesondere eine Aufsicht auf das Objekt, eine Durchsicht durch das Objekt oder einen Schnitt durch das Objekt repräsentiert und dieser zusammen mit wenigstens einem das Objekt in einer Projektion durch das Objekt hindurch repräsentierenden 2D-Datensatz oder einer zeitlichen Folge von 2D-Datensätzen mittels einer Bildwiedergabeeinheit wiedergegeben.

## Patentansprüche

1. Erfassungsvorrichtung (1) zum Erfassen eines Objekts (10) in bis zu drei Dimensionen,
mit einem Röntgensender (3), welcher ausgebildet ist Röntgenstrahlen (12) auszusenden und einem in einer Erfassungsebene angeordneten Detektor (5) für die Röntgenstrahlen (12), welcher derart angeordnet und ausgebildet ist, die Röntgenstrahlen (12) zu erfassen und wenigstens einen 2D-Datensatz zu erzeugen, welcher das Objekt (10) in einer Projektion durch das Objekt (10) hindurch auf die Erfassungsebene repräsentiert, wobei die Erfassungsvorrichtung (1) einen C-Bogen (9) aufweist, welcher mit dem Röntgensender (3) und mit dem Detektor (5) verbunden ist,
und die Erfassungsvorrichtung (1) ausgebildet ist, einen das Objekt (10) in drei räumlichen Dimensionen repräsentierenden 3D-Datensatz (19) aus einer Mehrzahl von 2D-Datensätzen (18) zu erzeugen, welche das Objekt (10) jeweils in zueinander verschiedenen Erfassungsrichtungen in einer Projektion durch das Objekt (10) hindurch repräsentieren und den 3D-Datensatz (19) in einem Speicher (17) vorrätig zu halten,
**dadurch gekennzeichnet,**
**dass** die Erfassungsvorrichtung (1) eine mit dem C-Bogen (9) wirkverbundene Stellvorrichtung (11) aufweist, welche ausgebildet ist, den C-Bogen (9) in Abhängigkeit von einem eingangsseitig empfangenen Stellsignal in wenigstens zwei oder drei rotatorischen Freiheitsgraden zu bewegen und in einer durch das Stellsignal repräsentierten Erfassungsposition festzustellen,
und die Erfassungsvorrichtung (1) ausgebildet ist, aus dem 3D-Datensatz (19) einen Bild-Datensatz zu erzeugen, welcher das Objekt, insbesondere eine Aufsicht auf das Objekt, eine Durchsicht durch das Objekt oder einen Schnitt durch das Objekt, repräsentiert und diesen zusammen mit dem wenigstens einen 2D-Datensatz zum Wiedergeben mittels wenigstens einer Bildwiedergabeeinheit (26) auszugeben,
und die Erfassungsvorrichtung (1) einen Positionsspeicher (25) für Positionsdatensätze (23) aufweist, welche jeweils eine Erfassungsposition repräsentieren und die Erfassungsvorrichtung (1) ausgebildet ist, in Abhängigkeit von einem Benutzerinteraktionssignal wenigstens einen Positionsdatensatz (23) aus dem Positionsspeicher (25) auszulesen und ein entsprechendes Stellsignal zum Bewegen des C-Bogens (9) in die Erfassungsposition zu erzeugen und in der Erfassungsposition mittels des Detektors (5) einen 2D-Datensatz (18) zu erzeugen.

2. Erfassungsvorrichtung nach Anspruch 1,
**dadurch gekennzeichnet,**
**dass** die Erfassungsvorrichtung (1) einen Ortsensor (28) aufweist, welcher ausgebildet ist, einen Instrumentort eines medizinischen Instruments (30, 30') in einem für das Erfassen des Objekts (10) vorgesehenen räumlichen Bereich zu erfassen, und einen den Instrumentort repräsentierenden Instrument-Datensatz zu erzeugen und diesen einem dem Instrumentort entsprechenden Bereich des 3D-Datensatzes (19) zuzuordnen und den Bilddatensatz in Abhängigkeit von dem Instrument-Datensatz derart zu erzeugen, dass der Bilddatensatz zusätzlich das medizinische Instrument (30') repräsentiert.

3. Erfassungsvorrichtung nach einem der vorhergehenden Ansprüche,
**dadurch gekennzeichnet,**
**dass** die Erfassungsvorrichtung einen Koordinatenspeicher (20) aufweist und ausgebildet ist, einen wenigstens einen Erfassungsort des 3D-Datensatzes (19) repräsentierenden Objekt-Koordinaten-Datensatz (22) zu erzeugen und den Objekt-Koordinaten-Datensatz (22) in dem Koordinatenspeicher (20) abzuspeichern, und den in dem Koordinatenspeicher (20) abgespeicherten Objekt-Koordinaten-Datensatz (22) auszulesen und den Instrumentort im Verhältnis zu dem ausgelesenen Objekt-Koordinaten-Datensatz (22) auszugeben.

4. Erfassungsvorrichtung nach einem der vorhergehenden Ansprüche,
**dadurch gekennzeichnet,**
**dass** die Erfassungsvorrichtung (1) eine Bildverarbeitungseinheit (24) aufweist, welche ausgebildet ist, aus dem 3D-Datensatz (19) einen 2D-Datensatz zu erzeugen, welcher eine Projektion durch das durch den 3D-Datensatz repräsentierte Objekt hindurch repräsentiert und diesen ausgangsseitig auszugeben.

5. Erfassungsvorrichtung nach einem der vorhergehenden Ansprüche,
**dadurch gekennzeichnet,**
**dass** die Erfassungsvorrichtung ausgebildet ist, mittels des Detektors (5) eine zeitliche Folge von 2D-Datensätzen zu erzeugen, welche jeweils zeitlich aufeinanderfolgende Erfassungsergebnisse des Objekts (10) repräsentieren.

6. Erfassungsvorrichtung nach einem der vorhergehenden Ansprüche,
**dadurch gekennzeichnet,**
**dass** die Bildverarbeitungseinheit (24) ausgebildet ist, wenigstens zwei 2D-Datensätze für entsprechende Erfassungsorte voneinander zu subtrahieren und einen ein Subtraktionsergebnis repräsentierenden Angio-2D-Datensatz (27) zu erzeugen.

7. Erfassungsvorrichtung nach einem der vorhergehenden Ansprüche,
**dadurch gekennzeichnet,**
**dass** die Erfassungsvorrichtung einen Bewegungssensor (16) aufweist, welcher ausgebildet ist ein Objektbewegen des Objekts (10) zu erfassen und ein Bewegungssignal zu erzeugen, welches das Objektbewegen repräsentiert und die Erfassungsvorrichtung ausgebildet ist, ein Abhängigkeit von dem Bewegungssignal einen 3D-Datensatz (19) zu erzeugen oder einen 2D-Datensatz aus dem 3D-Datensatz (19) zu erzeugen.

8. Erfassungsvorrichtung nach einem der vorhergehenden Ansprüche,
**dadurch gekennzeichnet,**
**dass** die Stellvorrichtung (11) ausgebildet ist, den C-Bogen (9) in Abhängigkeit von dem Stellsignal in wenigstens zwei oder drei translatorischen Freiheitsgraden zu bewegen und in einer durch das Stellsignal repräsentierten Erfassungsposition festzustellen.

9. Verfahren zum Erfassen eines Objekts (10) in bis zu drei Dimensionen mittels Röntgenstrahlen (12), bei welchem mittels eines Detektors (5) für die Röntgenstrahlen (12) eine Mehrzahl von 2D-Datensätzen erzeugt werden, wobei die 2D-Datensätze das Objekt jeweils in zueinander verschiedenen Erfassungsrichtungen in einer Projektion durch das Objekt hindurch repräsentieren und ein das Objekt in drei räumlichen Dimensionen repräsentierender 3D-Datensatz (19) aus den 2D-Datensätzen erzeugt wird, welcher das Objekt in drei Dimensionen repräsentiert,
und aus dem 3D-Datensatz (19) ein Bild-Datensatz erzeugt wird, welcher das Objekt (10), insbesondere eine Aufsicht auf das Objekt, eine Durchsicht durch das Objekt oder einen Schnitt durch das Objekt repräsentiert und dieser zusammen mit wenigstens einem von dem Detektor (5) erzeugtem, das Objekt (10) in einer Projektion durch das Objekt hindurch repräsentierenden 2D-Datensatz (18) oder einer zeitlichen Folge von 2D-Datensätzen (18) mittels einer Bildwiedergabeeinheit (26) wiedergegeben wird,
**dadurch gekennzeichnet,**
**dass** wenigstens zwei Positionsdatensätze (23) vorrätig gehalten werden, welche jeweils zueinander verschiedene Erfassungspositionen zum Erzeugen eines 2D-Datensatzes (18) repräsentieren und für die Positionsdatensätze (23) ein Erfassen des Objekts in Abhängigkeit von einem Benutzerinteraktionssignal entsprechend der von den Positionsdatensätzen jeweils repräsentierten Erfassungsposition erfolgt und in der Erfassungsposition mittels des Detektors der 2D-Datensatz (18) oder die zeitliche Folge von 2D-Datensätzen (18) erzeugt wird.

10. Verfahren nach Anspruch 9,
**dadurch gekennzeichnet,**
**dass** und ein Instrumentort eines medizinischen Instruments (30) in einem für das Erfassen des Objekts (10) vorgesehenen räumlichen Bereich erfasst wird, und ein den Instrumentort repräsentierenden Instrument-Datensatz erzeugt und einem dem Instrumentort entsprechenden Bereich des 3D-Datensatzes (19) zugeordnet wird,
und aus dem 3D-Datensatz (19) ein Bild-Datensatz erzeugt wird, welcher das Objekt (10), insbesondere eine Aufsicht auf das Objekt, eine Durchsicht durch das Objekt oder einen Schnitt durch das Objekt, zusammen mit dem Instrument (30) repräsentiert.

11. Verfahren nach einem der Ansprüche 9 oder 10, bei dem aus wenigstens zwei 2D-Datensätzen (18) mittels Subtraktion für entsprechende Erfassungsorte ein Subtraktionsergebnis erzeugt wird und ein dieses repräsentierender Angio-2D-Datensatz (27) erzeugt wird und dieser zusammen mit dem Bild-Datensatz mittels der Bildwiedergabeeinheit (26) wiedergegeben wird.
